# EUROPEAN PATENT SPECIFICATION

(11) **EP 4 010 473 B1**
(45) Date of publication and mention of the grant of the patent: **20.11.2024**
(21) Application number: 20852180.7
(22) Date of filing: 07.08.2020
(51) Int. Cl.: C12N 15/10, C12N 15/11, C12P 19/34, C12Q 1/68, C12Q 1/6809, C40B 30/04, C12Q 1/683

(54) **COMPOSITIONS AND METHODS FOR DETECTING METHYLATED DNA**
ZUSAMMENSETZUNGEN UND VERFAHREN FÜR DEN NACHWEIS VON METHYLIERTER DNA
COMPOSITIONS ET PROCÉDÉS DE DÉTECTION D'ADN MÉTHYLÉ

(30) Priority: 09.08.2019 US 201962884942 P
(43) Date of publication of application: 15.06.2022
(73) Proprietor: Battelle Memorial Institute, Columbus, OH 43201 (US)
(72) Inventor: SPURBECK, Rachel R., Columbus, Ohio 43201 (US)
(74) Representative: Grünecker Patent- und Rechtsanwälte PartG mbB
(86) International application number: PCT/US2020/045425
(87) International publication number: WO 2021/030194

(56) References cited:
- WO-A1-2005/085477
- WO-A1-2018/119301
- US-A1- 2008 254 453
- US-A1- 2008 254 453
- US-A1- 2010 240 064
- US-A1- 2011 076 726
- US-A1- 2012 208 193
- LUO GUAN-ZHENG ET AL: "Characterization of eukaryotic DNA N6-methyladenine by a highly sensitive restriction enzyme-assisted sequencing", NATURE COMMUNICATIONS, vol. 7, no. 1, 15 April 2016 (2016-04-15), XP093011914, Retrieved from the Internet <URL:https://www.ncbi.nlm.nih.gov/pmc/articles/PMC4835550/pdf/ncomms11301.pdf> DOI: 10.1038/ncomms11301
- MCINTYRE ALEXA B. R. ET AL: "Single-molecule sequencing detection of N6-methyladenine in microbial reference materials", NATURE COMMUNICATIONS, vol. 10, no. 1, 4 February 2019 (2019-02-04), XP093011917, Retrieved from the Internet <URL:https://www.ncbi.nlm.nih.gov/pmc/articles/PMC6362088/pdf/41467_2019_Article_8289.pdf> DOI: 10.1038/s41467-019-08289-9
- FU YE ET AL: "N6-Methyldeoxyadenosine Marks Active Transcription Start Sites inChlamydomonas", CELL, ELSEVIER, AMSTERDAM NL, vol. 161, no. 4, 30 April 2015 (2015-04-30), pages 879 - 892, XP029224302, ISSN: 0092-8674, DOI: 10.1016/J.CELL.2015.04.010

## Description

### CROSS REFERENCE TO RELATED APPLICATIONS

This application claims priority to U.S. Provisional Application Serial No. 62/884,942 filed on August 9, 2019.

### MATERIAL SUBMITTED ELECTRONICALLY

The present filing includes nucleotide/amino acid sequences in form of a sequence listing.

### BACKGROUND

Epigenetics is the study of heritable changes in gene expression that do not involve changes to the underlying primary nucleic acid sequence. Epigenetic change is a regular and natural occurrence but can also be influenced by several factors including age, the environment/lifestyle, and disease state. There is a renewed interest in epigenetics, as epigenic modifications have been associated with a host of disorders including various cancers, mental retardation associated disorders, immune disorders, neuropsychiatric disorders and pediatric disorders.

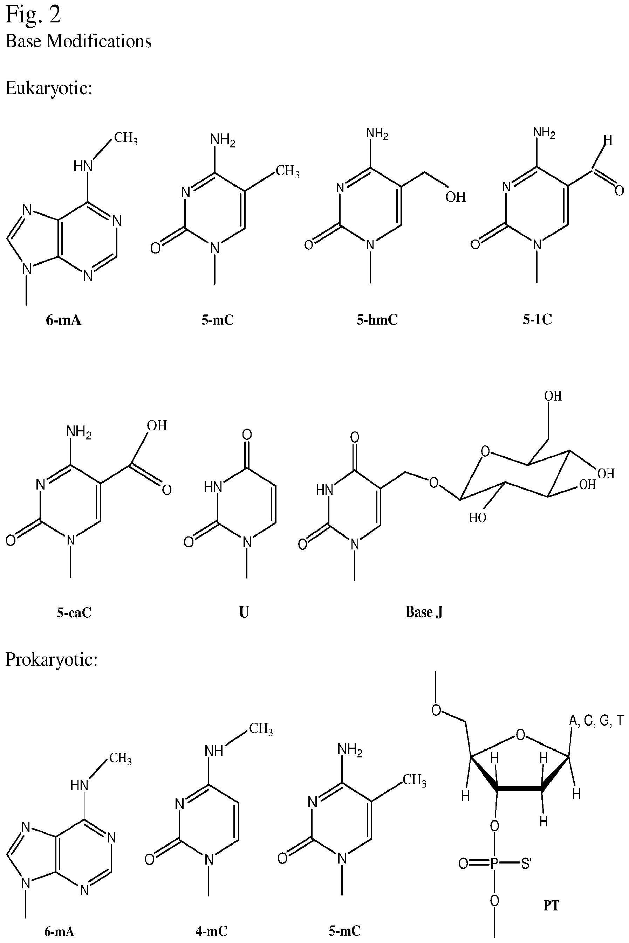

Epigenetic marks include DNA methylation, histone modifications, and regulatory RNA. Epigenetics is most often studied in multicellular organisms such as humans and plants, where epigenetic marks function in embryogenesis, cellular differentiation, genomic imprinting, and play roles in pathogenesis of diseases such as cancer. In bacteria, histones do not exist; therefore, non-coding RNAs and DNA methylation are the only universal epigenetic marks. The roles of DNA methylation in bacteria range from defense against bacteriophage infection, initiation of DNA replication, DNA repair, and gene regulation. 5N methylcytosine (5mC) is the most common epigenetic mark in higher eukaryotes, whereas 6N methyladenosine (6mA) is the most common epigenetic mark in bacteria. However, both marks are present in eukaryotes and prokaryotes, along with other nucleotide base modifications (See Fig. 2). Direct evidence has linked 6mA to several species of eubacteria, mosquitoes, wheat, protists, and indirect evidence links 6mA to several archaea and vertebrates. However, methods to determine the global 6mA patterns using common next generation sequencing (NGS) techniques are not commercially available. Accordingly, the role of 6mA in eukaryotic genomic function may be under appreciated due to a failure to detect 6mA.

In prokaryotes, 6mA not associated with restriction modification systems is produced by the action of two methyl transferases, deoxyadenosine methylase (DAM), which methylates at GATC sites, and DNA methylase N-4/N-6 domain-containing protein (CcrM), which methylates at the motif GANTC. Studies have shown that 6mA is present in the mitochondrial and chloroplast genomes, and therefore, could play a regulatory role throughout the Tree of Life.

WO 2005/085477 A1 describes methods for detecting the presence of methylation at a locus within a population of nucleic acids. Luo, Guan-Zheng et al. (Nature Comm. Vol. 7(1), 2016) describes the characterization of eukaryotic DNA N⁶-methyladenine by a highly sensitive restriction enzyme-assisted sequencing. While 5mC can be measured by conventional bisulfite sequencing, global 6mA patterns at the nucleotide level are currently detected only by PacBio Sequencing (Korlach, J. and S.W. Turner, Curr Opin Struct Biol, 2012. 22(3): p. 251-61), requiring a specialized, costly instrument and significant consumables to reach the depth of sequencing necessary for methylated base calls i.e., -$100,000 for a human genome). Thus there is a need for a new methodology that allows for the rapid and cost effective analysis of the presence of 6mA in a sample of genomic DNA.

In accordance with one embodiment of the present disclosure, applicant provides a novel method, "6mA-Seq", to identify 6mA residues by sequence analysis using an Illumina sequencer or equivalent equipment.

### SUMMARY

Currently, DNA methylation is one of the most broadly studied and well-characterized epigenetic modifications. Bacteria, like eukaryotes, use methylation to regulate gene expression. However, methylation profiling is not common in bacteria due to a lack of methodology pertinent to study of the dominant epigenetic marker in prokaryotes, 6N methyladenine (6 mA). In accordance with one embodiment of the present disclosure a method is provided for assessing global 6 mA profiles in bacterial genomes. The method can be used to monitor changes in methylation patterns of bacterial genomic DNA over time and/or in response to various environmental factors, including for example temperature, availability of nutrients and presence of stimulants or toxins.

In accordance with one embodiment a method is provided for monitoring global methylation patterns in genomic DNAs recovered from organisms or cell populations. More particularly, the method is directed to assessing the methylated state of genomic sequences associated with one or more target restriction enzyme recognition sites as further specified in the appended claims. In one embodiment the method of detecting methylated nucleic acid residues comprises the steps of first obtaining a library of genomic DNA and then subjecting the library of genomic DNA to restriction enzymatic digestion with either 1) enzymes that cut only at sites when the DNA is methylated (leaving a library enriched for unmethylated regions) or enzymes that cut only unmethylated regions (leaving a library enriched for methylated sequences). The DNA sequences of the restriction enzyme cleaved library are then analyzed using Next Generation Sequencing (NGS) to determine the sequences remaining in the restriction enzyme cleaved library, wherein comparison of the sequences identified by the Next Generation Sequencing step to a reference library of all available target restriction enzyme recognition sites present in relevant genome reveals those sites that were methylated in the analyzed sample.

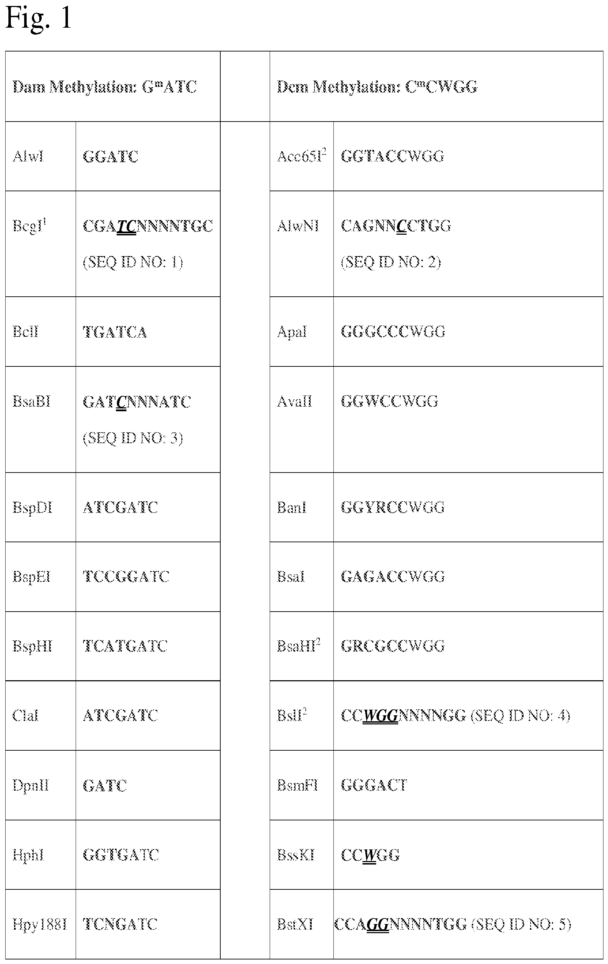

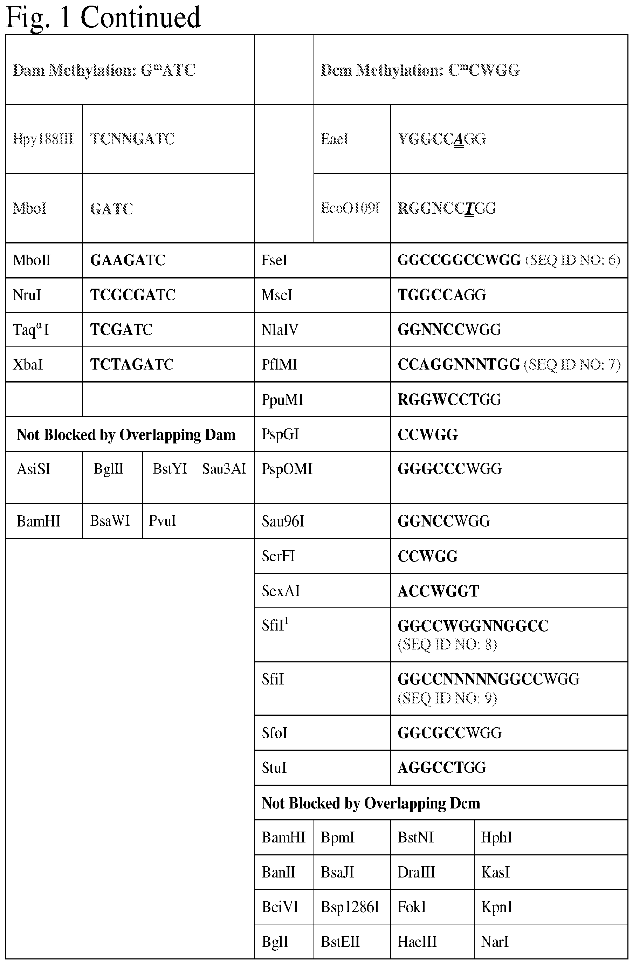

In one embodiment the method of detecting methylated nucleic acid residues in a sample comprising genomic DNA comprises the steps of first obtaining a library of genomic DNAs and then contacting the library with a methylation specific restriction enzyme that cleaves said target nucleic acid recognition site only when said target nucleic acid recognition site is unmethylated, to produce a set of digested genomic nucleic acid molecules enriched in methylated sequences. In one embodiment the methylation sensitive restriction enzyme is selected from the restriction enzymes listed in Fig. 1. In one embodiment the restriction enzyme used to digest the genomic DNA is selected from the group consisting of DpnI, DpnII and MboI.

The DNA sequences of the restriction enzyme cleaved library is then analyzed using Next Generation Sequencing to determine the sequences remaining in the restriction enzyme cleaved library. Comparison of the sequences identified by the Next Generation Sequencing step with a reference genomic sequences (said reference genomic sequence comprising all of the respective genomic sequence comprising the the restriction enzyme recognition site) reveals the unmethylated sequence in the analyzed genome as sequences missing relative in the restriction enzyme cleaved library relative to the reference genomic sequences. The sequences detected in the restriction enzyme cleaved library that comprise a target restriction enzyme recognition site are identified as methylated sequences.

In accordance with one embodiment the genomic DNA to be analyzed for the presence of methylated nucleotides (e.g., 6mA) is processed prior to enzymatic directions. More particularly, a PCR-Free library of genomic DNA is prepared suitable for Next Generation Sequencing. In one embodiment the preparation of the PCR-free library comprises the steps of isolating genomic DNA from a cell/ organism without an amplification step, fragmenting the isolated genomic DNA into DNA sequences less than 1 Kb in length, and typically having an average size of about 300 bp to about 600 bp, and the ligating the fragments of the genomic DNA to adapters that include all necessary components for sequencing primer annealing and attachment to a flow-cell surface for conducting Next-Generation Sequencing.

In one embodiment the step of restriction enzyme digestion of the genomic DNA is conducted using two different restriction enzymes that cleave the same recognition sequence but where one enzyme is sensitive to methylation and the other is not. The initial library of genomic DNA is divided into a first and second pool of genomic DNA wherein the first pool of genomic DNA is digested with a restriction enzyme that cleaves its target nucleic acid recognition site only when said target nucleic acid recognition site is unmethylated to produce a first set of digested genomic nucleic acid molecules (enriched in methylated DNAs), and second pool of genomic DNA is digested with a restriction enzyme that cleaves its target nucleic acid recognition site regardless of the methylated state of the target recognition site to produce a second set of digested genomic nucleic acid molecules. The sequence of the digested nucleic acids of the first and second digested genomic nucleic acid molecules is then determined, typically by using Next Generation Sequencing techniques. The nucleic acid sequences of the first and second digested genomic nucleic acid molecules are compared, and sequences present in the first set of digested genomic nucleic acid molecules relative to the second set of digested genomic nucleic acid molecules are identified as methylated sequences. In one embodiment the restriction enzyme used to digest the first pool of genomic DNA is selected from the group consisting of DpnI, DpnII and MboI and the restriction enzyme used to digest the second pool of genomic DNA is Sau3AI

### BRIEF DESCRIPTION OF THE DRAWINGS

Fig. 1 presents a table of known restriction enzymes that are methylation sensitive. Recognition sites appear in bold type. Nucleotides in addition to the recognition site that are required to produce an overlapping methylation site appears as normal type (not bold). Bases constrained by the requirements of an overlapping methylation site that would otherwise be degenerate (N, R, or Y) are indicated by italics and double underline red. For palindromic enzymes, both ends of the recognition sequence must be considered for possible overlapping methylation, e.g. ClaI is blocked by Dam methylation at GATCGAT and ATCGATC.
Fig. 2 presents a listing of the structure of methylated nucleotide bases found in eukaryotic and prokaryotic organisms.

### DETAILED DESCRIPTION

### DEFINITIONS

In describing and claiming the invention, the following terminology will be used in accordance with the definitions set forth below.

The term "about" as used herein means greater or lesser than the value or range of values stated by 10 percent, but is not intended to designate any value or range of values to only this broader definition. Each value or range of values preceded by the term "about" is also intended to encompass the embodiment of the stated absolute value or range of values.

As used herein, the term "purified" and like terms relate to the isolation of a molecule or compound in a form that is substantially free of contaminants normally associated with the molecule or compound in a native or natural environment.
As used herein, the term "purified" does not require absolute purity; rather, it is intended as a relative definition. The term "purified nucleic acid" is used herein to describe a nucleic acid which has been separated from other compounds including, but not limited to polypeptides, lipids and carbohydrates.

The term "isolated" requires that the referenced material be removed from its original environment (e.g., the natural environment if it is naturally occurring). For example, a naturally-occurring nucleic acid present in a living animal is not isolated, but the same nucleic acid, separated from some or all of the coexisting materials in the natural system, is isolated.

As used herein the terms "restriction endonuclease" or "restriction enzyme" are used interchangeably and encompass proteins that are able to cleave a double stranded DNA sequence at or near a specific sequence of nucleotides.

As used herein the term "restriction enzyme recognition site" defines locations on a DNA molecule containing a specific sequence of nucleotides that are recognized by the individual restriction enzyme and result in the cleavage of the sequence between two nucleotides within its recognition site, or somewhere nearby.

As used herein the term "methylation sensitive restriction enzyme" encompasses restriction enzymes whose cleavage is blocked or inhibited when the restriction enzyme recognition site is methylated by the cognate methylase.

### EMBODIMENTS

In accordance with one embodiment, the present disclosure is directed to a method for analyzing global methylation patterns in genomes, particularly in bacterial genomes or the genomes of eukaryotic organelles as further specified in the appended claims. Restriction enzymes that select for digestion of DNA at sites with differential methylation enable one to remove either unmethylated or methylated DNA and then compare the library that remains to a full genome to identify regions of methylation on a genome wide basis. Such methods can be used to establish correlations between certain methylation patterns and disease states or conditions, and/or determine the impact of various environmental factors on the methylated state of the genome and their corresponding impact on gene expression.

Restriction endonucleases are known that will only cleave their target recognition site when the DNA is in a unmethylated state. Advantageously, as disclosed herein such restriction enzymes can be used to detect the presence of methylated nucleic acids in genomic sequences, and more significantly analyze the methylated state of DNA sequences at genomic level.

Fig. 1 provides a list of restriction enzymes that are methylation sensitive. Restriction enzyme cleavage is blocked or substantially inhibited when the recognition sequence is methylated by the cognate methylase. More particularly, methylation of nucleic acid bases at or near the restriction enzyme recognition site can block cleavage, leave cleavage unaffected, or slow the rate or extent of cleavage. A restriction enzyme database, REBASE, is known to those skilled in the art for providing more detailed information regarding methylation sensitive restriction enzymes.

In accordance with the present disclosure, methylation sensitive restriction enzymes can be used to determine and monitor methylation patterns of genomic DNAs on a global level. In particular, one can track how methylation of genomic DNA sequences is altered by exposure to various environmental factors or genetic background. The methods disclosed herein can be used to analyze genomic DNA isolated from any cell, including organelle genomic DNA from chloroplasts and/or mitochondria of eukaryotic cells. In one embodiment the methylation state of genomic DNA isolated from bacterial cells is analyzed using the methods disclosed herein.

There are three common DNA methylases that are present in bacterial cells such as E. *coli*: the Dam methylase, Dcm methylase and EcoKI methylase. The methylase encoded by the dam gene (Dam methylase) transfers a methyl group from S-adenosylmethionine (SAM) to the N6 position of the adenine residues in the sequence GATC. The Dcm methylase (encoded by the dcm gene; referred to as the Mec methylase in earlier references) methylates the internal (second) cytosine residues in the sequences CCAGG and CCTGG at the C5 position. The EcoKI methylase, M. EcoKI, modifies adenine residues in the sequences AAC(N6)GTGC and GCAC(N6)GTT. EcoKI sites (~1 site per 8 kb) are much less common than Dam sites (~1 site per 256 bp) or Dcm sites (~1 site per 512 bp) in DNA of random sequence (GC=AT).

In one embodiment genomic DNA is isolated from a cell and subjected to enzymatic digestion using a methylation sensitive endonuclease. In one embodiment the isolated genomic DNA is first processed and a library of the genomic DNA is prepared from the processed genomic DNA. In accordance with one embodiment, the genomic DNA is first fractionated to reduce the average size of the genomic DNA prior to preparation of the library. The genomic DNA can be fractionated using any standard technique known to those skilled in the art to reduce the size of the average genomic DNA to less than 1 Kb in length. In one embodiment the DNA is fractionated by ultasonification and/or nebulization, including for example the use of the Covaris Adaptive Focused Acoustics (AFA) technology (Covaris, Inc., 14 Gill Street, Unit H, Woburn, Massachusetts, 01801-1721 USA) to generate fragments having an average size of about 350 bp to about 550 bp, or about 250 bp to about 350 bp. Ultasonification shearing can be used to generate double-stranded DNA (dsDNA) fragments with 3' or 5' overhangs (see US 9,103,755).

The fractionated DNA is then linked to the appropriate adapters to create a library of fractionated genomic DNA sequences. Preferably the creation of the genomic libraries is conducted in the absence of a PCR amplification step (i.e., a PCR-free library). In accordance with one embodiment the fragmented genomic DNA is subjected to a repair step wherein the overhangs resulting from fragmentation are converted into blunt ends. In one embodiment a 3' to 5' exonuclease activity removes the 3' overhangs, and a 5' to 3' polymerase activity completes the 5' overhangs. In a final step the fragments of genomic DNA are ligated to adapters that include all necessary components for sequencing primer annealing and attachment to a flow-cell surface for conducting Next-Generation Sequencing. See Kozarewa et al, Nature Methods 2009; 6:291-295; and Illumina TruSeq DNA PCR-Free Illumina, Inc. 5200 Illumina Way, San Diego, CA 92122 USA).

In accordance with one embodiment PCR-free genomic libraries are prepared from the cells whose genomic DNA will be assessed for methylation patterns. PCR amplification is commonly used in generating libraries for Next-Generation Sequencing (NGS) to efficiently enrich and amplify sequenceable DNA fragments. However, it introduces bias in the representation of the original complex template DNA. Accordingly, in one embodiment of the present invention libraries of genomic DNA will be prepared in the absence of a PCR amplification step and the digestion with methylation sensitive restriction enzymes will be conducted on the PCR-free library components.

In one embodiment a method is provided for analyzing 6mA methylation patterns in genomic DNA, including genomic DNA isolated from prokaryotics. The method comprises obtaining a PCR-Free library of genomic sequences and subjecting the library to at least one restriction enzyme digestion (cleaving DNA but only when the recognition site is unmethylated (e.g., lacking a 6mA residue). Optionally a second enzymatic digestion can be conducted wherein the second enzyme cleaves the same sites as the first but irregardless of whether the site is methylated or unmethylated. The digested library can optionally be amplified by PCR to enrich for uncut sequences. The enzymatic digested library sequence can be sequenced (typically by NGS) and compared to the corresponding reference sequences (comprising all known target recognition sites for the relevant genome) to determine where methylation was or was not, depending on the enzyme used. In one embodiment this analysis is conducted on bacterial DNA or other DNA to assess N6-methyladenosine patterns over time and/or and in relation to exposure to different environmental factors.

Bacteria primarily use 6mA for epigenetic regulation of gene expression. In accordance with one embodiment methods are provided for detecting 6mA in genomic DNA using Illumina platforms. In one embodiment, whole genome sequencing libraries will be prepared, polymerase chain reaction-free, from DNA from each organism or cell line. These libraries are treated with a mix of enzymes that remove library molecules that contain methylated adenosines (such as 6mA), leaving behind only molecules that do not have a methylated adenosine. The libraries will then be amplified to enrich the 6mA libraries for complete molecules and sequenced on the appropriate Illumina sequencing platform. Adapters and low-quality reads will be trimmed with Trimmomatic software prior to analysis of the data. Data analysis includes alignment to the reference sequence by BWA-MEM software and visualization through IGV genome viewer. A bed file of each genome will be produced with the coordinates of possible adenosine methylation sites. Coverage at these possible methylation sites will be determined and compared to the average coverage across the genome. Regions where reads are absent or more than two standard deviations below the mean in coverage represent regions where adenosine bases were not methylated. Analysis of methylated versus unmethylated sites in this manner will provide a global map of 6mA across the genome.

A list of methylation sensitive and insensitive restriction enzymes is provided in Fig. 1. In accordance with one embodiment the methylation sensitive restriction enzyme used in the present invention is selected from the group consisting of DpnI, DpnII and MboI. MboI, DpnI and DpnII each recognize and cleave at the recognition sequence GATC and both enzymes are blocked by dam methylation, but not blocked by dcm methylation. HinfI recognizes and cleaves at the recognition sequence GANTC, and cleavage is not blocked by either dam methylation, or dcm methylation. HpaII and MspI recognize and cleave at the recognition sequence CCGG recognition site and can be used for C5-methylcytosine detection.

The global analysis of 6mA in species beyond prokaryotes will revolutionize the field of epigenetics, demonstrating the impact of 6mA in response to environmental changes. State-of-the-art analysis methods overlook 6mA, although it is known to regulate processes in both prokaryotes and eukaryotes. It is now apparent that 6mA is an important regulator in all organisms but requires a lower limit of detection than possible back in the 1970s. The 6mA modification can regulate gene expression in specific organelles, including mitochondria which is targeted by environmental contaminants such as PFOA and causes a large impact on the lives of multicellular eukaryotes.

In accordance with the present disclosure a kit is provided for analyzing DNA for the presence of 6mA. The kit comprises one or more methylation sensitive restriction enzymes, optionally selected from the group consisting of DpnI, DpnII and MboI. The kit may contain additional reagents for preparing PCR-free libraries.

In accordance with a first embodiment, a method of detecting methylated nucleic acid residues, optionally a N6-methyladenosine (6mA) modification, in one or more target nucleic acid recognition sites present in a sample of genomic DNA is provided, wherein said method comprising the steps of
a) obtaining a library of genomic DNA as specified in the claims;
b) contacting said library with a methylation specific restriction enzyme that cleaves said target nucleic acid recognition site only when said target nucleic acid recognition site is unmethylated to produce a set of digested genomic nucleic acid molecules as further specified in the claims; and
c) analyzing the digested genomic nucleic acid molecules using next generation sequencing to determine the methylation state of said target nucleic acid recognition sites as further specified in the claims.

In accordance with another embodiment the library of genomic DNA of the first embodiment is prepared by isolating genomic DNA from a cell without a PCR amplification step;
fragmenting the genomic DNA to an average size of about 300 bp to about 600 bp;
ligating the fragmented genomic DNA to adapters wherein said adapter comprise a primer sequence for sequence analysis and optionally additional sequences complementary to sequences linked to a solid support.

In accordance with another embodiment, the method of the above embodiments is provided wherein the library of genomic DNA is contacted with a methylation specific restriction enzyme selected from the group consisting of DpnI, DpnII and MboI, optionally the library of genomic DNA is contacted with two or more methylation sensitive restriction enzymes.

In accordance with another embodiment, the method of any one of the above embodiments is provided wherein the genomic DNA is isolated from a prokaryote.

In accordance with another embodiment, the method of any one of the above embodiments is provided wherein the analyzing step comprises determining the nucleic acid sequence of said digested genomic nucleic acid molecules and comparing those nucleic acid sequences to a reference set of nucleic acids that represent all available target nucleic acid recognition sites present in said sample of genomic DNA, wherein sequences missing from the digested genomic nucleic acid molecules relative to said reference set represents a unmethylated target sequence in said sample of genomic DNA.

In accordance with a second embodiment, a method of detecting methylated nucleic acid residues, optionally a N6-methyladenosine (6mA) modification, in one or more target nucleic acid recognition sites present in genomic DNA is provided wherein, said method comprising the steps of
a) obtaining a library of genomic DNA fragments, wherein said genomic DNA fragments comprise isolated genomic DNAs that have been fragmented to have an average size of about 300 bp to about 600 bp, and have been further modified by covalent linkage of an adapter sequences that comprises a DNA sequencing primer to each DNA fragment, optionally wherein the library consists of genomic DNA fragments that have not been subjected to PCR;
b) contacting said library with a methylation specific restriction enzyme that cleaves said target nucleic acid recognition site, optionally wherein the target site comprises the sequence of GATC, only when said target nucleic acid recognition site is unmethylated to produce a set of digested genomic nucleic acid molecules as further specified in the claims; and
c) analyzing the digested genomic nucleic acid molecules using next generation sequencing to determine the methylation state of said target nucleic acid recognition sites as further specified in the claims.

In accordance with another embodiment, the method of the second embodiment is provided wherein the methylation specific restriction enzyme is selected from the group consisting of DpnI, DpnII and MboI.

In accordance with another embodiment, the method of the second embodiments above is provided wherein the genomic DNA fragments further comprise sequences complementary to sequences linked to a solid support.

In accordance with another embodiment, the method of any one of the second embodiments above is provided wherein the library of genomic DNA is contacted with two or more methylation sensitive restriction enzymes.

In accordance with another embodiment, the method of any one of the second embodiments above is provided wherein the genomic DNA is isolated from a prokaryote.

In accordance with another embodiment, the method of any one of the second embodiments above is provided wherein the analyzing step comprises determining the nucleic acid sequence of said digested genomic nucleic acid molecules and comparing those nucleic acid sequences to a reference set of nucleic acids that represent all available target nucleic acid recognition sites present in said sample of genomic DNA, wherein sequences missing from the digested genomic nucleic acid molecules relative to said reference set represents a unmethylated target sequence in said sample of genomic DNA.

In accordance with another embodiment, the method of any one of the second embodiments above is provided wherein the library of genomic DNA of step a) is divided into a first and second pool of genomic DNA, and said method comprises the steps of
contacting said first pool of genomic DNA with first restriction enzyme that cleaves said target nucleic acid recognition site only when said target nucleic acid recognition site is unmethylated, to produce a first set of digested genomic nucleic acid molecules;
contacting said second pool of genomic DNA with a second restriction enzyme that cleaves said target nucleic acid recognition site in the presence or absence methylation, to produce a second set of digested genomic nucleic acid molecules, with the proviso that the first and second restriction enzymes each have the same nucleic acid recognition site;
determining the nucleic acid sequence of the first and second digested genomic nucleic acid molecules using Next Generation Sequencing;
comparing the nucleic acid sequences of the first digested genomic nucleic acid molecules to the nucleic acid sequences of second digested genomic nucleic acid molecules; and
identifying nucleic acid sequences present in the first digested genomic nucleic acid molecules that are missing in the second digested genomic nucleic acid molecules as methylated sequences.

In accordance with another embodiment, the method of any one of the second embodiments above is provided wherein the target nucleic acid recognition site comprises a nucleic acid sequence of GATC or GANTC, optionally wherein the target nucleic acid recognition site comprises a nucleic acid sequence of GATC.

In accordance with a third embodiment, a method of detecting genomic sequences comprising N6-methyladenosine (6mA) is provided wherein said method comprises the steps of
a) obtaining a library of genomic DNA fragments, wherein said genomic DNA fragments comprise isolated genomic DNAs that have not been subjected to PCR, have been fragmented to have an average size of about 300 bp to about 600 bp and have been further modified by covalent linkage of an adapter sequences that comprises a DNA sequencing primer to each DNA fragment;
b) contacting the genomic DNA fragments of said library with a methylation sensitive restriction enzyme that cannot cleave its recognition site when 6mA is present in the restriction site or within 1 or 2 nucleotides of said recognition site to produce a restriction enzyme cleaved library comprising a set of digested genomic nucleic acid molecules and uncut methylated nucleic acid molecules;
c) obtaining the sequence of the uncut methylated nucleic acid molecules; and
d) analyzing the sequence data generated in step c) to identify sequences as being unmethylated when the sequence is not detected relative to a reference library of sequences known to be present in said library of genomic DNA fragments and comprising the recognition site of said methylation sensitive restriction enzyme.

In accordance with another embodiment, the method of the third embodiment is provided wherein the genomic DNA of said library is contacted with two or more methylation sensitive restriction enzymes.

In accordance with another embodiment, the method of any one of the third embodiments above is provided wherein the methylation sensitive restriction enzyme is selected from the group consisting of MboI, DpnI and DpnII.

In accordance with another embodiment, the method of any one of the third embodiments above is provided wherein the genomic DNA is prokaryotic DNA.

In accordance with another embodiment, the method of any one of the third embodiments above is provided wherein the library of genomic DNA fragments is prepared by
isolating genomic DNA from prokaryotic cells without a PCR amplification step;
fragmenting the genomic DNA to an average size of about 300 bp to about 600 bp;
ligating the fragmented genomic DNA to adapters wherein said adapters comprise a primer sequence for sequence analysis and optionally additional sequences complementary to sequences linked to a solid support.

In accordance with another embodiment, the method of any one of the third embodiments above is provided wherein the library of genomic DNA of step a) is divided into a first and second pool of genomic DNA, and said method comprises the steps of
contacting the first pool of genomic DNA with a first restriction enzyme that cannot cleave its recognition site when 6mA is present in the restriction site or within 1 or 2 nucleotides of said recognition site, to produce a first set of digested genomic nucleic acid molecules;
contacting the second pool of genomic DNA with a second restriction enzyme that cleaves said target nucleic acid recognition site in the presence or absence methylation, to produce a second set of digested genomic nucleic acid molecules, with the proviso that the first and second restriction enzymes each have the same nucleic acid recognition site; and
determining the nucleic acid sequence of the first and second digested genomic nucleic acid molecules using Next Generation Sequencing; and
comparing the nucleic acid sequences of the first digested genomic nucleic acid molecules to the nucleic acid sequences of second digested genomic nucleic acid molecules; and
identifying nucleic acid sequences present in the first digested genomic nucleic acid molecules that are missing in the second digested genomic nucleic acid molecules as sequences containing 6mA residues.

In accordance with another embodiment, the method of any one of the third embodiments above is provided wherein the methylation sensitive restriction enzyme is selected from the group consisting of DpnI, DpnII and MboI.

In accordance with another embodiment, the method of any one of the third embodiments above is provided wherein the first restriction enzyme is selected from the group consisting of DpnI, DpnII and MboI and the second restriction enzyme is Sau3AI.

### EXAMPLE 1

### Identification of 6mA in genomic sequences

A proof of concept study was conducted using two strains of *Escherichia coli.* One strain was derived from *E. coli K-12* with the genotype *dam⁻*/*dcm⁻,* causing it to be unable to methylate adenosine or cytosine. The other strain was *E. coli* ATCC 25922, which is wild-type *dam*⁺/*dcm*⁺*,* enabling it to methylate its genome at both cytosine and adenosine. PCR-free Illumina sequencing libraries were prepared from DNA from both strains. These libraries were then treated with enzymes that remove library sequences that contained unmethylated adenosines leaving behind only sequences that contained potential 6mA sites. The *dam⁻*/*dcm⁻* strain had 19146 possible 6mA sites of which none were methylated according to the 6mA sequencing analysis, whereas the *dam*⁺/*dcm*⁺ strain had 98.8% of its 20460 possible 6mA sites methylated (Table 1). This data demonstrates the utility of the 6mA-Seq method for detection of differential 6mA.

**Table 1. 6mA sequencing coverage statistics for dam⁻/dcm⁻ and dam⁺/dcm⁺ E. coli strains**

| ***dam*/*dcm* (+/-)** | **# of possible 6mA sites** | **Total genome coverage** | **Coverage at possible 6mA sites** | **% methylated** |
|---|---|---|---|---|
| -/- | 19146 | 61.8 | 0 | 0% |
| -/- | 19146 | 10.7 | 0 | 0% |
| +/+ | 20460 | 49.3 | 46 | 98.8% |
| +/+ | 20460 | 16.5 | 16 | 98.8% |

This method does not require any specialized equipment, utilizes a protocol that is very similar to whole genome sequencing on the Illumina platform, and costs the same as whole genome sequencing. Therefore, the method is easy to use by any lab with access to an Illumina sequencer. The current method should be expandable to detect differential 6mA methylomes across the Tree of Life as the enzymes used to target 6mA will work on any 6mA methylated DNA with the same efficiency.

### Impact of differential culture conditions on global methylation

Two *Escherichia coli* strains, E coli ATCC 25922 with both dam and dcm activity and *E coli* K 12 with genotype dam-/dcm- were cultured in different media. DNA from each strain was extracted for further analysis using methylation specific restriction enzymes and next generation sequencing. Data analysis consisted in evaluating methylation patterns to find unmethylated locations as absence of sequencing reads (See Table 2).

218 GATC sites demonstrated hyper or hypomethylation in *E*. *coli* ATCC25922. 3 GATC loci had consistent hypomethylation in all four growth conditions (position start: 777328, 3928044, and 5032297), whereas 2 were consistently hypermethylated (position start: 2907398 and 2907498). 9 GATC sites (position start: 1661619, 2581408, 2581441, 2581801, 2582072, 2582134, 2718715, 2906872, and 2907142) were consistently hypermethylated in M9 minimal media regardless of carbon source and were not in LB. 9 GATC sites were hypomethylated only in LB (position start: 409277, 479355, 1579192, 1684645, 2181531, 2498588, 2957634, 3782914, and 4031299). LB shared 6 hypomethylated GATC sites with M9-glycerol and M9-glucose (position start: 665850, 786885, 3782985, 3928100, 5031607, and 5032236).

The data demonstrates the utility and high sensitivity of 6 mA Seq for detection of differentially methylated loci in bacteria. While this method is limited by the identification of methylation at the restriction enzyme motifs, the analysis can be expanded by the use of additional methylation sensitive enzymes to digest the library of genomic sequences, thus analyzing methylation at motifs beyond GATC, which will increase the diversity of epigenetic signatures that can be identified by this technique.

As demonstrated 6 mA Seq analytical technique readily identifies sites with differential methylation patterns. *E coli* which cannot methylate adenine has no coverage at GATC sites, whereas *E coli* with DAM methylase has 98.8% of sites methylated
6 mA hyper and hypomethylation status changes in different growth conditions at loci across the genome.

LB grown *E coli* cultures are very different in methylation pattern from those in M9 minimal media, with only 5 GATC sites consistently hyper or hypomethylated between all conditions tested. The 3 hypomethylated sites correspond to a biosynthetic threonine ammonia lyase, a tRNA Ala and a location approximately 100 bp from this tRNA Ala. The two hypermethylated sites correspond to IS 3 family transposase and
ClbS/DfsB family four helix bundle protein.

In each culture condition there were unique *E coli* hyper or hypomethylated sites compared to other conditions tested.

| Table 2. Differentially methylated GATC sites by culture media | | |
|---|---|---|
| **Culture Media** | | |
| | **# Hypermethylated GATCs (% Unique)** | **# Hypomethylated GATCs (% Unique)** |
| **LB** | **2 (0%)** | **25 (36%)** |
| **M9-sorbitol** | **73 (64%)** | **7 (43%)** |
| **M9-glucose** | **88 (63%)** | **39 (59%)** |
| **M9-glycerol** | **41 (51%)** | **22 (27%)** |
| **LB or M9-sorbitol** | **0** | **0** |
| **LB or M9-glucose** | **0** | **4** |
| **LB or M9-glycerol** | **0** | **3** |
| **M9-sorbitol or M9-glucose** | **14** | **0** |
| **M9-sorbitol or M9-glycerol** | **1** | **1** |
| **M9-glucose or M9-glycerol** | **8** | **3** |
| **LB, M9-glycose, or M9-glycerol** | **0** | **6** |
| **M9 independent of carbon source** | **9** | **0** |
| **All** | **2** | **3** |

## Claims

1. A method of detecting methylated nucleic acid residues in one or more target nucleic acid recognition sites present in genomic DNA, said method comprising the steps of
a) obtaining a library of genomic DNA fragments, wherein said genomic DNA fragments comprise isolated genomic DNAs that have not been subjected to PCR, have been fragmented to have an average size of about 300 bp to about 600 bp and have been further modified by covalent linkage of an adapter sequences to each DNA fragment wherein the adapter sequence comprises a DNA sequencing primer;
b) contacting said library with a methylation specific restriction enzyme that cleaves said target nucleic acid recognition site only when said target nucleic acid recognition site is unmethylated to produce a restriction enzyme cleaved library comprising a set of digested genomic nucleic acid molecules and uncut methylated nucleic acid molecules; and
c) analyzing the restriction enzyme cleaved library using next generation sequencing to determine the nucleic acid sequence of said uncut methylated genomic nucleic acid molecules and comparing those nucleic acid sequences to a reference set of nucleic acids that represent all available target nucleic acid recognition sites present in said library of genomic DNA fragments wherein sequences missing from the restriction enzyme cleaved library relative to said reference set of nucleic acids represent unmethylated target sequence in said sample of genomic DNA.

2. The method of claim 1, wherein the methylation specific restriction enzyme is selected from the group consisting of Dpnl, DpnII and Mbol, optionally wherein the library of genomic DNA is contacted with two or more methylation sensitive restriction enzymes.

3. The method of any one of claims 1 to 2, wherein said genomic DNA fragments further comprise sequences complementary to sequences linked to a solid support.

4. The method of any one of claims 1 to 3, wherein the genomic DNA is isolated from a prokaryote.

5. The method of any one of claims 1 to 4, further comprising the steps of dividing the library of genomic DNA of step a) into a first and second pool of genomic DNA, and said method comprises the steps of
contacting said first pool of genomic DNA with first restriction enzyme that cleaves said target nucleic acid recognition site only when said target nucleic acid recognition site is unmethylated, to produce a first set of digested genomic nucleic acid molecules;
contacting said second pool of genomic DNA with a second restriction enzyme that cleaves said target nucleic acid recognition site in the presence or absence methylation, to produce a second set of digested genomic nucleic acid molecules, with the proviso that the first and second restriction enzymes each have the same nucleic acid recognition site;
determining the nucleic acid sequence of the first and second digested genomic nucleic acid molecules using Next Generation Sequencing;
comparing the nucleic acid sequences of the first digested genomic nucleic acid molecules to the nucleic acid sequences of second digested genomic nucleic acid molecules; and
identifying nucleic acid sequences present in the first digested genomic nucleic acid molecules that are missing in the second digested genomic nucleic acid molecules as methylated sequences.

6. The method of any one of claims 1 to 5, wherein the target nucleic acid recognition site comprises a nucleic acid sequence of GATC or GANTC.

7. The method of claim 5 or 6, wherein the first restriction enzyme is selected from the group consisting of Dpnl, DpnII and MboI and the second restriction enzyme is Sau3AI.

8. A method of detecting genomic sequences comprising N6-methyladenosine (6mA) within a target nucleic acid recognition site or within 1 or 2 nucleotides of said target nucleic acid recognition site, said method comprising the steps of
a) obtaining a library of genomic DNA fragments, wherein said genomic DNA fragments comprise isolated genomic DNAs that have not been subjected to PCR, have been fragmented to have an average size of about 300 bp to about 600 bp and have been further modified by covalent linkage of an adapter sequences to each DNA fragment wherein the adapter sequence comprises a DNA sequencing primer;
b) contacting the genomic DNA fragments of said library with a methylation sensitive restriction enzyme that cannot cleave said target nucleic acid recognition site when 6mA is present in the restriction site or within 1 or 2 nucleotides of said recognition site to produce a restriction enzyme cleaved library comprising a set of digested genomic nucleic acid molecules and uncut methylated nucleic acid molecules;
c) obtaining the sequence of the uncut methylated nucleic acid molecules; and
d) analyzing the sequence data generated in step c) to identify sequences as being unmethylated when the sequence is not detected relative to a reference library of sequences known to be present in said library of genomic DNA fragments and comprising the recognition site of said methylation sensitive restriction enzyme.

9. The method of claim 8, wherein the genomic DNA of said library is contacted with two or more methylation sensitive restriction enzymes.

10. The method of claim 8, wherein the methylation sensitive restriction enzyme is selected from the group consisting of MboI, DpnI and DpnII.

11. The method of any one of claims 8 to 10, wherein said genomic DNA is prokaryotic DNA.

12. The method of claim 8, wherein the library of genomic DNA fragments is prepared by
isolating genomic DNA from prokaryotic cells without a PCR amplification step;
fragmenting the genomic DNA to an average size of about 300 bp to about 600 bp;
ligating the fragmented genomic DNA to adapters wherein said adapters comprise a primer sequence for sequence analysis and optionally additional sequences complementary to sequences linked to a solid support.

13. The method of any one of claims 8 to 12, further comprising the steps of dividing the library of genomic DNA of step a) into a first and second pool of genomic DNA, and said method comprises the steps of
contacting the first pool of genomic DNA with a first restriction enzyme that cannot cleave said target nucleic acid recognition site when 6mA is present in the restriction site or within 1 or 2 nucleotides of said recognition site, to produce a first set of digested genomic nucleic acid molecules;
contacting the second pool of genomic DNA with a second restriction enzyme that cleaves said target nucleic acid recognition site in the presence or absence methylation, to produce a second set of digested genomic nucleic acid molecules, with the proviso that the first and second restriction enzymes each have the same nucleic acid recognition site; and
determining the nucleic acid sequence of the first and second digested genomic nucleic acid molecules using Next Generation Sequencing; and
comparing the nucleic acid sequences of the first digested genomic nucleic acid molecules to the nucleic acid sequences of second digested genomic nucleic acid molecules; and
identifying nucleic acid sequences present in the first digested genomic nucleic acid molecules that are missing in the second digested genomic nucleic acid molecules as sequences containing 6mA residues.

14. The method of any one of claims 8 to 13, wherein the methylation sensitive restriction enzyme is selected from the group consisting of DpnI, DpnII and MboI.

15. The method of claim 13, wherein the first restriction enzyme is selected from the group consisting of DpnI, DpnII and MboI and the second restriction enzyme is Sau3AI.

## Patentansprüche

1. Ein Verfahren zum Nachweis von methylierten Nukleinsäureresten in einer oder mehreren Ziel-Nukleinsäure-Erkennungsstellen, die in genomischer DNA vorhanden sind, wobei das Verfahren die folgenden Schritte umfasst:
a) Erhalten einer Bibliothek genomischer DNA-Fragmente, wobei die genomischen DNA-Fragmente isolierte genomische DNAs umfassen, die nicht einer PCR unterzogen wurden, die so fragmentiert wurden, dass sie eine durchschnittliche Größe von etwa 300 bp bis etwa 600 bp aufweisen, und die ferner durch kovalente Bindung einer Adaptersequenz an jedes DNA-Fragment modifiziert wurden, wobei die Adaptersequenz einen DNA-Sequenzierungsprimer umfasst;
b) In-Kontakt-Bringen der Bibliothek mit einem methylierungsspezifischen Restriktionsenzym, das die Ziel-Nukleinsäure-Erkennungsstelle nur dann spaltet, wenn die Ziel-Nukleinsäure-Erkennungsstelle unmethyliert ist, um eine durch Restriktionsenzym gespaltene Bibliothek herzustellen, die einen Satz von verdauten genomischen Nukleinsäuremolekülen und ungeschnittenen methylierten Nukleinsäuremolekülen umfasst; und
c) Analysieren der durch Restriktionsenzym gespaltenen Bibliothek unter Verwendung von Next-Generation-Sequenzierung, um die Nukleinsäuresequenz der ungeschnittenen methylierten genomischen Nukleinsäuremoleküle zu bestimmen, und Vergleichen dieser Nukleinsäuresequenzen mit einem Referenzsatz von Nukleinsäuren, die alle verfügbaren Ziel-Nukleinsäure-Erkennungsstellen repräsentieren, die in der Bibliothek von genomischen DNA-Fragmenten vorhanden sind, wobei Sequenzen, die in der durch Restriktionsenzym gespaltenen Bibliothek im Vergleich zu dem Referenzsatz von Nukleinsäuren fehlen, eine unmethylierte Zielsequenz in der Probe von genomischer DNA repräsentieren.

2. Verfahren nach Anspruch 1, wobei das methylierungsspezifische Restriktionsenzym aus der Gruppe bestehend aus Dpnl, DpnII und Mbol ausgewählt ist, wobei die Bibliothek genomischer DNA gegebenenfalls mit zwei oder mehr methylierungssensitiven Restriktionsenzymen in Kontakt gebracht wird.

3. Verfahren nach einem der Ansprüche 1 bis 2, wobei die genomischen DNA-Fragmente ferner Sequenzen umfassen, die zu an einen festen Träger gebundenen Sequenzen komplementär sind.

4. Verfahren nach einem der Ansprüche 1 bis 3, wobei die genomische DNA aus einem Prokaryoten isoliert ist.

5. Verfahren nach einem der Ansprüche 1 bis 4, ferner umfassend die Schritte des Aufteilens der genomischen DNA-Bibliothek aus Schritt a) in einen ersten und einen zweiten Pool genomischer DNA, und wobei das Verfahren die Schritte umfasst des
In-Kontakt-Bringens des ersten Pools genomischer DNA mit einem ersten Restriktionsenzym, das die Ziel-Nukleinsäure-Erkennungsstelle nur dann spaltet, wenn die Ziel-Nukleinsäure-Erkennungsstelle unmethyliert ist, um einen ersten Satz verdaute genomische Nukleinsäuremoleküle herzustellen;
In-Kontakt-Bringens des zweiten Pools genomischer DNA mit einem zweiten Restriktionsenzym, das die Ziel-Nukleinsäure-Erkennungsstelle in Gegenwart oder Abwesenheit von Methylierung spaltet, um einen zweiten Satz verdaute genomische Nukleinsäuremoleküle zu erzeugen, mit der Maßgabe, dass das erste und das zweite Restriktionsenzym jeweils dieselbe Nukleinsäure-Erkennungsstelle aufweisen;
Bestimmens der Nukleinsäuresequenz der ersten und zweiten verdauten genomischen Nukleinsäuremoleküle unter Verwendung von Next-Generation-Sequenzierung;
Vergleichens der Nukleinsäuresequenzen der ersten verdauten genomischen Nukleinsäuremoleküle mit den Nukleinsäuresequenzen der zweiten verdauten genomischen Nukleinsäuremoleküle; und
Identifizierens von Nukleinsäuresequenzen, die in den ersten verdauten genomischen Nukleinsäuremolekülen vorhanden sind und in den zweiten verdauten genomischen Nukleinsäuremolekülen fehlen, als methylierte Sequenzen.

6. Verfahren nach einem der Ansprüche 1 bis 5, wobei die Ziel-Nukleinsäure-Erkennungsstelle eine Nukleinsäuresequenz von GATC oder GANTC umfasst.

7. Verfahren nach Anspruch 5 oder 6, wobei das erste Restriktionsenzym aus der Gruppe bestehend aus Dpnl, DpnII und Mbol ausgewählt ist und das zweite Restriktionsenzym Sau3AI ist.

8. Verfahren zum Nachweis von genomischen Sequenzen, die N6-Methyladenosin (6mA) innerhalb einer Ziel-Nukleinsäure-Erkennungsstelle oder innerhalb von 1 oder 2 Nukleotiden der Ziel-Nukleinsäure-Erkennungsstelle umfassen, wobei das Verfahren die folgenden Schritte umfasst:
a) Erhalten einer Bibliothek genomischer DNA-Fragmente, wobei die genomischen DNA-Fragmente isolierte genomische DNAs umfassen, die nicht einer PCR unterzogen wurden, die so fragmentiert wurden, dass sie eine durchschnittliche Größe von etwa 300 bp bis etwa 600 bp aufweisen, und die ferner durch kovalente Bindung einer Adaptersequenz an jedes DNA-Fragment modifiziert wurden, wobei die Adaptersequenz einen DNA-Sequenzierungsprimer umfasst;
b) In-Kontakt-Bringen der genomischen DNA-Fragmente der Bibliothek mit einem methylierungssensitiven Restriktionsenzym, das die Ziel-Nukleinsäure-Erkennungsstelle nicht spalten kann, wenn 6mA in der Restriktionsstelle oder innerhalb von 1 oder 2 Nukleotiden der Erkennungsstelle vorhanden ist, um eine durch Restriktionsenzym gespaltene Bibliothek herzustellen, die einen Satz von verdauten genomischen Nukleinsäuremolekülen und ungeschnittenen methylierten Nukleinsäuremolekülen umfasst;
c) Erhalten der Sequenz der ungeschnittenen methylierten Nukleinsäuremoleküle; und
d) Analysieren der in Schritt c) erzeugten Sequenzdaten, um Sequenzen als unmethyliert zu identifizieren, wenn die Sequenz nicht relativ zu einer Referenzbibliothek von Sequenzen nachgewiesen wird, von denen bekannt ist, dass sie in der Bibliothek genomischer DNA-Fragmente vorhanden sind, und die die Erkennungsstelle des methylierungssensitiven Restriktionsenzyms umfassen.

9. Verfahren nach Anspruch 8, wobei die genomische DNA der Bibliothek mit zwei oder mehr methylierungssensitiven Restriktionsenzymen in Kontakt gebracht wird.

10. Verfahren nach Anspruch 8, wobei das methylierungssensitive Restriktionsenzym aus der Gruppe ausgewählt wird, die aus Mbol, Dpnl und DpnII besteht.

11. Verfahren nach einem der Ansprüche 8 bis 10, wobei die genomische DNA prokaryotische DNA ist.

12. Verfahren nach Anspruch 8, wobei die Bibliothek genomischer DNA-Fragmente hergestellt wird durch
Isolieren genomischer DNA aus prokaryotischen Zellen ohne einen PCR-Amplifikationsschritt;
Fragmentieren der genomischen DNA auf eine durchschnittliche Größe von etwa 300 bp bis etwa 600 bp;
Ligieren der fragmentierten genomischen DNA an Adapter, wobei die Adapter eine Primersequenz für die Sequenzanalyse und gegebenenfalls zusätzliche Sequenzen, die zu an einen festen Träger gebundenen Sequenzen komplementär sind, umfassen.

13. Verfahren nach einem der Ansprüche 8 bis 12, ferner umfassend die Schritte des Aufteilens der Bibliothek genomischer DNA von Schritt a) in einen ersten und zweiten Pool genomischer DNA, und wobei das Verfahren die Schritte umfasst des
In-Kontakt-Bringens des ersten Pools genomischer DNA mit einem ersten Restriktionsenzym, das die Ziel-Nukleinsäure-Erkennungsstelle nicht spalten kann, wenn 6mA in der Restriktionsstelle oder innerhalb von 1 oder 2 Nukleotiden der Erkennungsstelle vorhanden ist, um einen ersten Satz verdaute genomische Nukleinsäuremoleküle herzustellen;
In-Kontakt-Bringens des zweiten Pools genomischer DNA mit einem zweiten Restriktionsenzym, das die Ziel-Nukleinsäure-Erkennungsstelle in Gegenwart oder Abwesenheit von Methylierung spaltet, um einen zweiten Satz von verdauten genomischen Nukleinsäuremolekülen herzustellen, mit der Maßgabe, dass das erste und das zweite Restriktionsenzym jeweils dieselbe Nukleinsäure-Erkennungsstelle aufweisen; und
Bestimmens der Nukleinsäuresequenz der ersten und zweiten verdauten genomischen Nukleinsäuremoleküle unter Verwendung von Next-Generation Sequenzierung; und
Vergleichens der Nukleinsäuresequenzen der ersten verdauten genomischen Nukleinsäuremoleküle mit den Nukleinsäuresequenzen der zweiten verdauten genomischen Nukleinsäuremoleküle; und
Identifizierens von Nukleinsäuresequenzen, die in den ersten verdauten genomischen Nukleinsäuremolekülen vorhanden sind und in den zweiten verdauten genomischen Nukleinsäuremolekülen fehlen, als Sequenzen, die 6mA-Reste enthalten.

14. Verfahren nach einem der Ansprüche 8 bis 13, wobei das methylierungssensitive Restriktionsenzym aus der Gruppe bestehend aus Dpnl, DpnII und Mbol ausgewählt ist.

15. Verfahren nach Anspruch 13, wobei das erste Restriktionsenzym aus der Gruppe bestehend aus Dpnl, DpnII und Mbol ausgewählt ist und das zweite Restriktionsenzym Sau3AI ist.

## Revendications

1. Procédé de détection de résidus d'acide nucléique méthylés dans un ou plusieurs sites de reconnaissance d'acide nucléique cible présents dans de l'ADN génomique, ledit procédé comprenant les étapes consistant à
a) obtenir une bibliothèque de fragments d'ADN génomique, dans lequel lesdits fragments d'ADN génomique comprennent des ADN génomiques isolés qui n'ont pas fait l'objet d'une PCR, ont été fragmentés pour avoir une taille moyenne d'environ 300 bp à environ 600 bp et ont été en outre modifiés par liaison covalente de séquences adaptatrices à chaque fragment d'ADN, dans lequel la séquence adaptatrice comprend une amorce de séquençage d'ADN ;
b) mettre en contact ladite bibliothèque avec une enzyme de restriction spécifique à la méthylation qui clive ledit site de reconnaissance d'acide nucléique cible uniquement lorsque ledit site de reconnaissance d'acide nucléique cible est non méthylé afin de produire une bibliothèque clivée par l'enzyme de restriction comprenant un ensemble de molécules d'acide nucléique génomique digérées et de molécules d'acide nucléique méthylées non coupées ; et
c) analyser la bibliothèque clivée par l'enzyme de restriction au moyen d'un séquençage de nouvelle génération afin de déterminer la séquence d'acide nucléique desdites molécules d'acide nucléique méthylées non coupées et de comparer ces séquences d'acide nucléique à un ensemble d'acides nucléiques de référence qui représentent tous les sites de reconnaissance d'acide nucléique cible disponibles présents dans ladite bibliothèque de fragments d'ADN génomique, dans lequel des séquences absentes de la bibliothèque clivée par l'enzyme de restriction par rapport audit ensemble d'acides nucléiques de référence représentent la séquence cible non méthylée dans ledit échantillon d'ADN génomique.

2. Procédé selon la revendication 1, dans lequel l'enzyme de restriction spécifique à la méthylation est choisie dans le groupe constitué par Dpnl, DpnII et Mbol, dans lequel, éventuellement, la bibliothèque d'ADN génomique est mise en contact avec deux enzymes de restriction sensibles à la méthylation ou plus.

3. Procédé selon l'une quelconque des revendications 1 à 2, dans lequel lesdits fragments d'ADN génomique comprennent en outre des séquences complémentaires de séquences liées à un support solide.

4. Procédé selon l'une quelconque des revendications 1 à 3, dans lequel l'ADN génomique est isolé à partir d'un procaryote.

5. Procédé selon l'une quelconque des revendications 1 à 4, comprenant en outre les étapes consistant à diviser la bibliothèque d'ADN génomique de l'étape a) en un premier et un second groupe d'ADN génomique, et ledit procédé comprend les étapes consistant à
mettre en contact ledit premier groupe d'ADN génomique avec une première enzyme de restriction qui clive ledit site de reconnaissance d'acide nucléique cible uniquement lorsque ledit site de reconnaissance d'acide nucléique cible est non méthylé, afin de produire un premier ensemble de molécules d'acide nucléique génomique digérées ;
mettre en contact ledit second groupe d'ADN génomique avec une seconde enzyme de restriction qui clive ledit site de reconnaissance d'acide nucléique cible en présence ou en l'absence de méthylation, afin de produire un second ensemble de molécules d'acide nucléique génomique digérées, à condition que les première et seconde enzymes de restriction aient chacune le même site de reconnaissance d'acide nucléique ;
déterminer la séquence d'acide nucléique des premières et secondes molécules d'acide nucléique digérées au moyen d'un séquençage de nouvelle génération ;
comparer les séquences d'acide nucléique des premières molécules d'acide nucléique digérées aux séquences d'acide nucléique des secondes molécules d'acide nucléique digérées ; et
identifier les séquences d'acide nucléique présentes dans les premières molécules d'acide nucléique digérées qui sont absentes des secondes molécules d'acide nucléique digérées comme étant des séquences méthylées.

6. Procédé selon l'une quelconque des revendications 1 à 5, dans lequel le site de reconnaissance d'acide nucléique cible comprend une séquence d'acide nucléique GATC ou GANTC.

7. Procédé selon la revendication 5 ou 6, dans lequel la première enzyme de restriction est choisie dans le groupe constitué par Dpnl, DpnII et Mbol et la seconde enzyme de restriction est Sau3Al.

8. Procédé de détection de séquences génomiques comprenant de la N6-méthyladénosine (6mA) au sein d'un site de reconnaissance d'acide nucléique cible ou au sein d'1 ou 2 nucléotides dudit site de reconnaissance d'acide nucléique cible, ledit procédé comprenant les étapes consistant à
a) obtenir une bibliothèque de fragments d'ADN génomique, dans lequel lesdits fragments d'ADN génomique comprennent des ADN génomiques isolés qui n'ont pas fait l'objet d'une PCR, ont été fragmentés pour avoir une taille moyenne d'environ 300 bp à environ 600 bp et ont été en outre modifiés par liaison covalente de séquences adaptatrices à chaque fragment d'ADN, dans lequel la séquence adaptatrice comprend une amorce de séquençage d'ADN ;
b) mettre en contact les fragments d'ADN génomique de ladite bibliothèque avec une enzyme de restriction sensible à la méthylation qui ne peut pas cliver ledit site de reconnaissance d'acide nucléique cible quand de la 6mA est présente dans le site de restriction ou à l'intérieur d'1 ou 2 nucléotides dudit site de reconnaissance afin de produire une bibliothèque clivée par l'enzyme de restriction comprenant un ensemble de molécules d'acide nucléique génomique digérées et de molécules d'acide nucléique méthylées non coupées ;
c) obtenir la séquence des molécules d'acide nucléique méthylées non coupées ; et
d) analyser les données de séquence générées à l'étape c) afin d'identifier les séquences comme étant non méthylées lorsque la séquence n'est pas détectée par rapport à une bibliothèque de référence de séquences connues pour être présentes dans ladite bibliothèque de fragments d'ADN génomique et comprenant le site de reconnaissance de ladite enzyme de restriction sensible à la méthylation.

9. Procédé selon la revendication 8, dans lequel l'ADN génomique de ladite bibliothèque est mis en contact avec deux enzymes de restriction sensibles à la méthylation ou plus.

10. Procédé selon la revendication 8, dans lequel l'enzyme de restriction sensible à la méthylation est choisie dans le groupe constitué par Mbol, Dpnl et DpnII.

11. Procédé selon l'une quelconque des revendications 8 à 10, dans lequel ledit ADN génomique est de l'ADN procaryotique.

12. Procédé selon la revendication 8, dans lequel la bibliothèque de fragments d'ADN génomique est préparée en
isolant de l'ADN génomique à partir de cellules procaryotiques sans étape d'amplification par PCR ;
fragmentant l'ADN génomique jusqu'à une taille moyenne d'environ 300 bp à environ 600 bp ;
liant l'ADN génomique fragmenté à des adaptateurs, dans lequel lesdits adaptateurs comprennent une séquence d'amorce pour l'analyse de séquences et éventuellement des séquences supplémentaires complémentaires de séquences liées à un support solide.

13. Procédé selon l'une quelconque des revendications 8 à 12, comprenant en outre les étapes consistant à diviser la bibliothèque d'ADN génomique de l'étape a) en un premier et un second groupe d'ADN génomique, et ledit procédé comprend les étapes consistant à
mettre en contact le premier groupe d'ADN génomique avec une première enzyme de restriction qui ne peut pas cliver ledit site de reconnaissance d'acide nucléique cible quand de la 6mA est présente dans le site de reconnaissance ou au sein d'1 ou 2 nucléotides dudit site de reconnaissance, afin de produire un premier ensemble de molécules d'acide nucléique génomique digérées ;
mettre en contact le second groupe d'ADN génomique avec une seconde enzyme de restriction qui clive ledit site de reconnaissance d'acide nucléique cible en présence ou en l'absence de méthylation, afin de produire un second ensemble de molécules d'acide nucléique génomique digérées, à condition que les première et seconde enzymes de restriction aient chacune le même site de reconnaissance d'acide nucléique ; et
déterminer la séquence d'acide nucléique des premières et secondes molécules d'acide nucléique digérées au moyen d'un séquençage de nouvelle génération ; et
comparer les séquences d'acide nucléique des premières molécules d'acide nucléique digérées aux séquences d'acide nucléique des secondes molécules d'acide nucléique digérées ; et
identifier les séquences d'acide nucléique présentes dans les premières molécules d'acide nucléique digérées qui sont absentes des secondes molécules d'acide nucléique digérées comme étant des séquences contenant des résidus de 6mA.

14. Procédé selon l'une quelconque des revendications 8 à 13, dans lequel l'enzyme de restriction sensible à la méthylation est choisie dans le groupe constitué par Dpnl, DpnII et Mbol.

15. Procédé selon la revendication 13, dans lequel la première enzyme de restriction est choisie dans le groupe constitué par Dpnl, DpnII et Mbol et la seconde enzyme de restriction est Sau3AI.
